# EUROPEAN PATENT SPECIFICATION

(11) **EP 3 339 810 B1**
(45) Date of publication and mention of the grant of the patent: **14.07.2021**
(21) Application number: 17208285.1
(22) Date of filing: 19.12.2017
(51) Int. Cl.: G01D 1/14, G01D 1/18, A61B 5/00, H04L 29/08

(54) **DETECTING TRIGGER CONDITIONS TO DYNAMICALLY UPDATE A MODEL BASED ON SENSOR DATA IN A FOG COMPUTING ENVIRONMENT**
DETEKTION VON AUSLÖSUNGSKONDITIONEN ZUR DYNAMISCHEN AKTUALISIERUNG EINES MODELLS AUF BASIS VON SENSORDATEN IN EINER FOG-COMPUTING-UMGEBUNG
DÉTECTION DE CONDITIONS DE DÉCLENCHEMENT POUR LA MISE À JOUR DYNAMIQUE D'UN MODÈLE BASÉ SUR DES DONNÉES DE CAPTEUR DANS UN ENVIRONNEMENT INFORMATIQUE DE BROUILLARD

(30) Priority: 23.12.2016 IN 201641044000; 27.11.2017 US 201715822883
(43) Date of publication of application: 27.06.2018
(73) Proprietor: Accenture Global Solutions Limited, Dublin 4 (IE)
(72) Inventor: MAITRA, Anutosh, 560076 Bangalore, Karnataka (IN); KUNTAGOD, Nataraj, 560093 Bangalore, Karnataka (IN); KUMARESAN, Senthil Kumar, 560048 Bangalore, Karnataka (IN); SRINIVAS SAI, Satya, 560037 Bangalore, Karnataka (IN)
(74) Representative: Müller-Boré & Partner Patentanwälte PartG mbB

(56) References cited:
- WO-A1-03/088830
- WO-A2-2011/091268
- US-A1- 2013 006 151
- US-A1- 2013 245 401
- US-A1- 2013 338 630
- US-A1- 2015 371 522
- US-A1- 2016 183 792
- US-A1- 2016 287 076
- Hermann Winner ET AL: "Handbuch Fahrerassistenzsysteme: Grundlagen, Komponenten und Systeme für aktive Sicherheit und Komfort" In: "Handbuch Fahrerassistenzsysteme: Grundlagen, Komponenten und Systeme für aktive Sicherheit und Komfort", 20 April 2015 (2015-04-20), Springer, XP055506347, ISBN: 978-3-658-05733-6 pages ToC,Ch14-Ch21,Ch26-Ch29,Ch38,,

## Description

### BACKGROUND

The Internet of things (IoT) may refer to a network of devices that interact without human intervention. In some cases, an IoT device (e.g., a sensor) may have reduced processing and/or storage capabilities, and may rely on another device in the network to perform resource-intensive tasks (e.g., expensive processing requests, bulk data storage, etc.).

US 2013/006151 A1 relates to a method for modelling pressure exposure and/or the risk of pressure ulcer formation. The method includes the steps of using pressure sensors to derive pressure exposure or risk values and displaying the pressure exposure or risk values in a graphical manner to a user. Computer-implemented systems includes a pressure-sensing interface mat and components for implementing the steps of the methods.

US 2016/287076 A1 relates to an infant data aggregation system. The system includes a platform interface and a platform processor. A platform interface is configured to receive measurement data from multiple infant monitoring systems, each of the multiple infant monitoring systems including an infant monitoring device and an infant monitoring hub. Each of the plurality of infant monitoring systems is associated with a corresponding infant. A platform processor is configured to remove personally identifiable information associated with the inferences received in order to generate infant de-identified inferences and analyze the inferences received from the plurality of infant monitoring systems in order to generate a dynamic model reflecting infant trends relating to the inference for infants of different ages. The dynamic model is viewable by users associated with infant monitoring systems. The platform interface may be further configured to send the dynamic model to a first infant monitoring system.

WO 03/088830 A1 relates to a patient monitoring system which provides a wireless communication link between a patient monitoring device, worn by a patient, and a local hub. The patient monitoring system is adapted to monitor various patient physiological characteristics, such as blood pressure, pulse rate, blood glucose, weight, pulse oximetry and others. The data from the patient monitoring device is wirelessly transmitted to a local hub, which, in turn, is configured to automatically transfer the data to a remote server, for example, over a public or private communications network. The server can be configured as a web portal to selectively allow access to such patient physiological data by designated third parties, such as physicians, clinicians, relatives and the patient themselves.

WO 2011/091268 A2 relates to a computer-implemented method and system for assisting a plurality of patients to manage chronic health conditions. The method, for each patient, comprises: (a) receiving information from the patient or a member of a patient care network on an expected patient activity at a given future time period; (b) determining expected transient local ambient conditions in the patient's surroundings during the expected patient activity at the given future time period; (c) predicting health exacerbations for the patient using a stored computer model of the patient based on a desired patient control set-point range, the expected patient activity, and the expected transient local ambient conditions; and (d) proactively sending a message to the patient or a member of the patient care network before the given future time period, the message alerting the patient or a member of the patient care network of the predicted health exacerbations for the patient and identifying one or more corrective actions for the patient to avoid or mitigate the predicted health exacerbations.

Thus, according to an aspect, the problem relates to how to improve updating a model applied by a device for analysing physiological data.

### SUMMARY

This problem is solved by the subject-matter of the independent claims. Preferred embodiments are defined in the dependent claims.

### BRIEF DESCRIPTION OF THE DRAWINGS

Fig. 1 is a diagram of an overview of an example implementation described herein;
Fig. 2 is a diagram of an example environment in which systems and/or methods, described herein, may be implemented;
Fig. 3 is a diagram of example components of one or more devices of Fig. 2; and
Fig. 4 is a flow chart of an example process for dynamically updating a model based on detecting a trigger condition.

### DETAILED DESCRIPTION

The following detailed description of example implementations refers to the accompanying drawings. The same reference numbers in different drawings may identify the same or similar elements.

Fog computing may refer to a decentralized network architecture where devices near an end-user (e.g., edge nodes) carry out processing and/or data storage capabilities that, in a more traditional network architecture, may be carried out by a cloud server (e.g., a network utilizing cloud computing). For example, in a network with a more traditional architecture, a sensor device may transmit sensor data to a cloud server, and the cloud server may analyze (e.g., process, store, monitor, etc.) the sensor data. In a network with a decentralized architecture, a sensor device may transmit sensor data to an edge node that is in close physical proximity to the sensor device, and the edge node may analyze the sensor data, while only periodically transmitting the sensor data to a cloud server. In some cases, the edge node may use a model (e.g., a conceptual model, a data model, a logical model, etc.) to analyze the sensor data in a particular manner. However, network changes that are not accounted for by the model (e.g., a sensor device may be updated to measure a different parameter, to measure for a different entity, etc.) may cause the model to analyze the sensor data inaccurately or may cause the model to be unable to analyze the sensor data.

Implementations described herein allow an edge node to analyze sensor data using a model, detect sensor data that is different from that which the model is configured to analyze, and dynamically update the model (e.g., by communicating with a cloud server) based on detecting changes to the sensor data. By dynamically updating the model based on changes to the sensor data, the edge node efficiently utilizes network resources. For example, the edge node may process and/or store sensor data when the sensor data can be analyzed by the model, and the edge node may transmit processing and/or storage requests to a cloud server when the sensor data cannot be analyzed by the model. Furthermore, in the case of physiological data, dynamically updating the model may permit real-time adaptation to rapidly changing patient conditions, may permit compliance with changing diagnostic requirements, and/or the like.

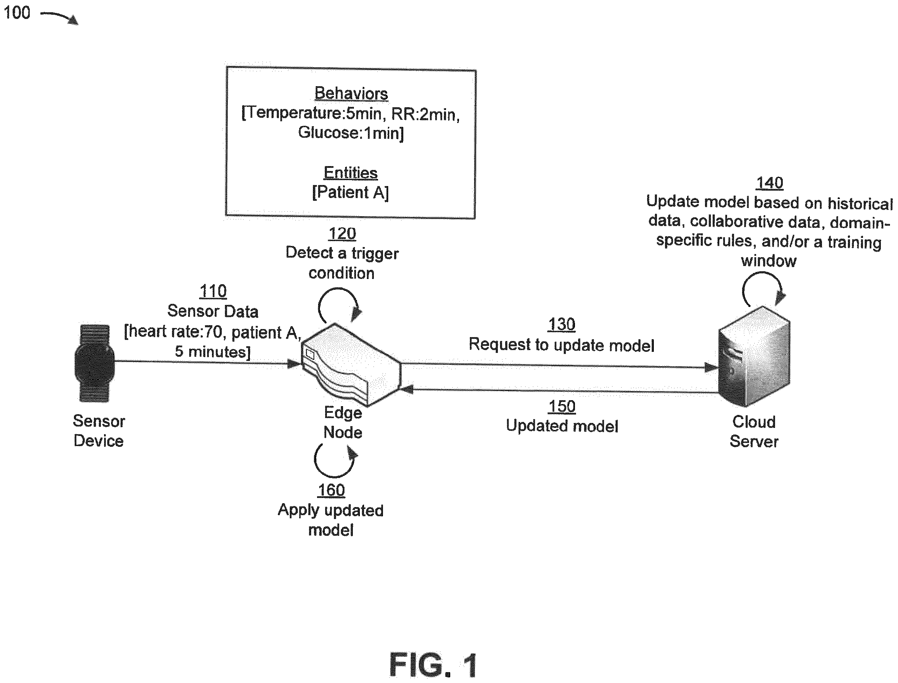

Fig. 1 is a diagram of an overview of an example implementation 100 described herein. For Fig. 1, assume a decentralized IoT system includes a sensor device (e.g., shown as a smart watch), an edge node, and a cloud server, and that the decentralized IoT system dynamically analyzes sensor data using a model. Further assume that the model is configured to analyze sensor data for one entity (e.g., patient A), that the sensor data includes information relating to three measureable parameters of the human body (e.g., shown as body temperature, which is measured every five minutes, respiratory rate (RR), which is measured every two minutes, and glucose level, which is measured every one minute).

As shown in Fig. 1, and by reference number 110, the edge node may receive the sensor data from the sensor device, and the sensor data may include information that identifies a parameter measurement (e.g., a heart rate of 70 beats per minute), information that identifies an entity associated with the parameter (e.g., patient A), information that identifies a frequency at which to measure the parameter (e.g., a heart rate may be measured every 5 minutes), or the like.

As shown by reference number 120, the edge node may detect a trigger condition by detecting a change in the sensor data. For example, the model applied by the edge node may analyze parameters of body temperature, respiratory rate, and glucose level, and receiving sensor data that includes information identifying a heart rate may indicate a change in the sensor data. In this case, the model applied by the edge node may be insufficient to analyze the information identifying the heart rate, which, as shown by reference number 130, may cause the edge node to transmit, to a cloud server, a request to update the model. Furthermore, the edge node may increase a frequency at which the sensor data is transmitted to the cloud server, which may provide the cloud server with additional sensor data that can be used to update the model faster than providing sensor data at a lower frequency (e.g., the edge node may request, from the sensor device, sensor data every minute instead of every five minutes). While the trigger condition is described as a change in sensor data for the purpose of Fig. 1, in some implementations, the trigger condition may include a change in the entity, a change in the frequency with which the edge node receives sensor data from sensor device(s), and/or the like.

As shown by reference number 140, the cloud server may update the model based on historical data, collaborative data, a set of domain-specific rules, a training window, and/or the like, as described in more detail elsewhere herein. For example, the cloud server may update the model with information relating to monitoring a heart rate by searching a data structure for historical data relating to patient A (e.g., to determine if patient A has a history of past heart rate measurements). If historical data is not found, the cloud server may search the data structure for collaborative data relating to patients with similar characteristics (e.g., to obtain heart rate information regarding patients of similar age, health, demographics, etc.).

Additionally, or alternatively, the cloud server may verify the accuracy of the sensor data used to update the model by comparing the sensor data received from the edge node to the domain-specific rules. For example, a domain-specific rule relating to monitoring a heart rate may provide a range of human heart rates, and a heart rate level outside of the range may indicate an error or an improper measurement, and may not be used to update the model. In some implementations, a domain-specific rule may include a constraint and/or function to be satisfied before updating the model (e.g., a time constraint in an industrial or manufacturing context, or the like). Furthermore, the cloud server may determine a training window, which may indicate a time period to be used to train (e.g., update) the model. In some cases, the cloud server may receive frequent requests to update the model. This may indicate to the cloud server that the model needs to be updated quickly. In other cases, the cloud server may receive infrequent requests to update the model, and this may indicate to the cloud server that the model does not need to be updated quickly. The cloud server may adjust the training window accordingly. In this way, the cloud server conserves network resources by increasing a frequency at which sensor data is received only when the cloud server regularly receives requests to update the model.

As shown by reference number 150, the edge node may receive, from the cloud server, the updated model. For example, the edge node may receive the updated model, which may include one or more functions capable of analyzing the sensor data. As shown by reference number 160, the edge node may apply the updated model to the sensor data and/or to subsequent sensor data received from the sensor device, which may allow the edge node to properly analyze the sensor data. By using the cloud server to update the model while continuing to analyze the sensor data that coincides with the current model, the edge node conserves processing resources and/or memory resources.

As indicated above, Fig. 1 is provided merely as an example. Other examples are possible and may differ from what was described with regard to Fig. 1. For example, while implementations described in Fig. 1 involve updating a model, other implementations may involve generating a new model. Additionally, or alternatively, while the decentralized IoT system described in Fig. 1 uses a sensor device, an edge node, and a cloud server, other implementations may include multiple sensor devices (e.g., hundreds, thousands, or billions of sensor devices), multiple edge nodes, and/or multiple cloud servers. In practice, a particular edge node may process thousands, millions, billions, trillions, or more instances of sensor data. Implementations are described herein as using particular types of sensor data. In practice, however, other types of sensor data may be used.

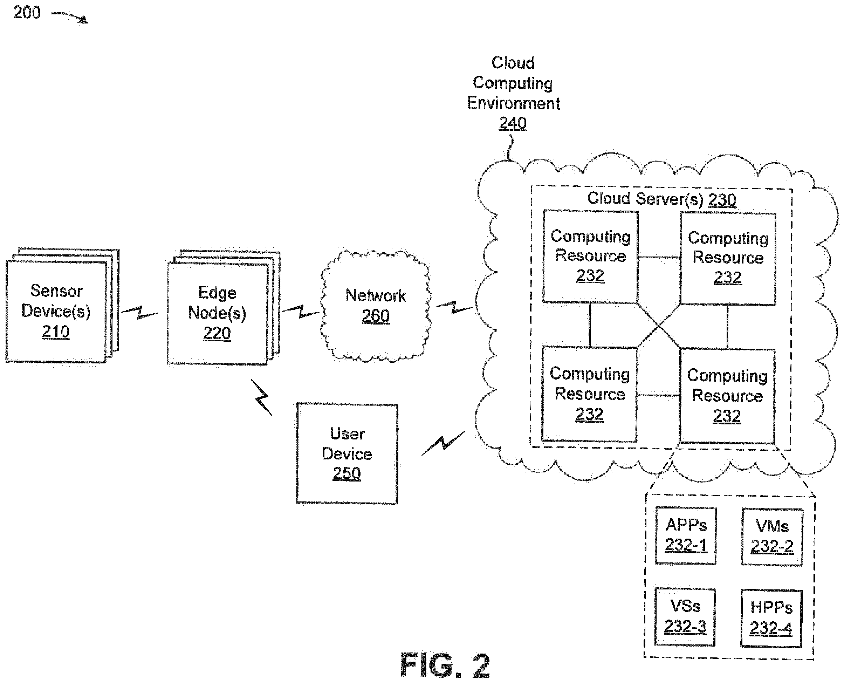

Fig. 2 is a diagram of an example environment 200 in which systems and/or methods, described herein, may be implemented. As shown in Fig. 2, environment 200 may include a set of sensor devices 210, a set of edge nodes 220, a set of cloud servers 230 hosted within a cloud computing environment 240, a user device 250, and a network 260. Devices of environment 200 may interconnect via wired connections, wireless connections, or a combination of wired and wireless connections.

Sensor device 210 includes one or more devices capable of detecting and/or measuring sensor data, and transmitting the sensor data to edge node 220. In some implementations, sensor device 210 may include a heart rate monitor, a blood pressure sensor, a glucose monitor, a pulse monitor, an accelerometer, a pedometer, a gyroscope, a heat flux sensor, a skin conductivity sensor, a temperature sensor (e.g., a skin temperature sensor, an air temperature sensor, etc.), a calorie monitor, a sleep monitor, a motion sensor, a moisture sensor (e.g., a perspiration sensor), a chemical sensor or chemical compound sensor (e.g., to measure oxygen, carbon dioxide, lactate, testosterone, cortisol, glucose, glucagon, glycogen, insulin, starch, free fatty acid, triglycerides, monoglycerides, glycerol, pyruvate, lipids, other carbohydrates, ketone bodies, choline), a microphone (e.g., to detect noises from the stomach, a burp, passing gas, noises from a bathroom, etc.), and/or the like. Additionally, or alternatively, sensor device 210 may include a sensor to measure sensor data associated with a physical environment, such as a geographic location, a speed, an acceleration, a time of day, an amount of sunlight, an air temperature level, a humidity level, an oxygen level, a carbon monoxide level, a moisture level, a wind level, and/or the like. In some implementations, sensor device 210 may be configured to automatically transmit the sensor data to edge node 220 after detecting and/or measuring a parameter, after detecting and/or measuring a set of parameters (i.e., to transmit a batch of the sensor data), or at a scheduled time.

Edge node 220 includes one or more devices capable of receiving, detecting, analyzing (e.g., storing, generating, processing, etc.) and/or providing sensor data. For example, edge node 220 may include a router, a gateway, a set-top box, an access point, a switch, a server, a desktop computer, a laptop computer, or a similar type of device. In some implementations, edge node 220 may include a node in a fog computing environment. In some implementations, edge node 220 may apply a model to sensor data received from sensor device 210, may detect a trigger condition associated with the sensor data, and may transmit a request, to cloud server 230, to update the model. Edge node 220 may have technical limitations, such as limited memory resources, limited processing resources, and/or the like, that prevent edge node 220 from storing and/or processing large amounts of data. For example, edge node 220 may be implemented as an embedded system (e.g., a system attached to a patient's body). Thus, edge node 220 may not be capable of analyzing large amounts of data, executing a complicated model, dynamically updating the model, and/or the like. Such analysis, execution, and dynamic updates may be beneficial to permit real-time processing of sensor data, such as physiological data that can change rapidly.

Cloud server 230 includes one or more devices capable of receiving, analyzing, and/or providing sensor data. For example, cloud server 230 may include a server, a group of servers, a desktop computer, a laptop computer, or the like. In some implementations, cloud server 230 may store historical data and/or collaborative data, data indicating of a set of domain-specific rules, data that may be used to determine a training window (e.g., a period during which to train a model), or the like. Additionally, or alternatively, cloud server 230 may use the stored data to update a model.

Cloud computing environment 240 includes an environment that hosts cloud server 230. Cloud computing environment 240 may provide computation, software, data access, storage, and/or other services that do not require end-user knowledge of a physical location and configuration of system(s) and/or device(s) that host cloud server 230. As shown, cloud computing environment 240 may include a group of computing resources 232 (referred to collectively as "computing resources 232" and individually as "computing resource 232").

Computing resource 232 includes one or more personal computers, workstation computers, server devices, or another type of computation and/or communication device. In some implementations, computing resource 232 may host cloud server 230. The cloud resources may include compute instances executing in computing resource 232, storage devices provided in computing resource 232, data transfer devices provided by computing resource 232, etc. In some implementations, computing resource 232 may communicate with other computing resources 232 via wired connections, wireless connections, or a combination of wired and wireless connections.

As further shown in Fig. 2, computing resource 232 includes a group of cloud resources, such as one or more applications ("APPs") 232-1, one or more virtual machines ("VMs") 232-2, virtualized storage ("VSs") 232-3, one or more hypervisors ("HYPs") 232-4, or the like.

Application 232-1 includes one or more software applications that may be provided to or accessed by edge node 220. Application 232-1 may eliminate a need to install and execute the software applications on edge node 220. For example, application 232-1 may include software associated with cloud server 230 and/or any other software capable of being provided via cloud computing environment 240. In some implementations, one application 232-1 may send/receive information to/from one or more other applications 232-1, via virtual machine 232-2.

Virtual machine 232-2 includes a software implementation of a machine (e.g., a computer) that executes programs like a physical machine. Virtual machine 232-2 may be either a system virtual machine or a process virtual machine, depending upon use and degree of correspondence to any real machine by virtual machine 232-2. A system virtual machine may provide a complete system platform that supports execution of a complete operating system ("OS"). A process virtual machine may execute a single program, and may support a single process. In some implementations, virtual machine 232-2 may manage infrastructure of cloud computing environment 240, such as data management, synchronization, or long-duration data transfers. In some implementations, computing resource 232 may include one or more physical servers instead of or in addition to one or more virtual machines 232-2.

Virtualized storage 232-3 includes one or more storage systems and/or one or more devices that use virtualization techniques within the storage systems or devices of computing resource 232. In some implementations, within the context of a storage system, types of virtualizations may include block virtualization and file virtualization. Block virtualization may refer to abstraction (or separation) of logical storage from physical storage so that the storage system may be accessed without regard to physical storage or heterogeneous structure. The separation may permit administrators of the storage system flexibility in how the administrators manage storage for end users. File virtualization may eliminate dependencies between data accessed at a file level and a location where files are physically stored. This may enable optimization of storage use, server consolidation, and/or performance of non-disruptive file migrations.

Hypervisor 232-4 provides hardware virtualization techniques that allow multiple operating systems (e.g., "guest operating systems") to execute concurrently on a host computer, such as computing resource 232. Hypervisor 232-4 may present a virtual operating platform to the guest operating systems, and may manage the execution of the guest operating systems. Multiple instances of a variety of operating systems may share virtualized hardware resources.

User device 250 includes one or more devices capable of communicating with edge node 220. For example, user device 250 may include a computing device, such as a desktop computer, a laptop computer, a mobile device (e.g., a mobile phone), a tablet computer, a wearable computer (e.g., a smart watch, a smart band, or a smart pair of eyeglasses), a server, or the like. In some implementations, user device 250 may receive sensor data from edge node 220, and/or may transmit the sensor data to cloud server 230.

Network 260 includes one or more wired and/or wireless networks. For example, network 260 may include a cellular network (e.g., a fifth generation (5G) network, a fourth generation (4G) network, such as a long-term evolution (LTE) network, a third generation (3G) network, a code division multiple access (CDMA) network, etc.), a public land mobile network (PLMN), a local area network (LAN), a wide area network (WAN), a metropolitan area network (MAN), a telephone network (e.g., the Public Switched Telephone Network (PSTN)), a private network, an ad hoc network, an intranet, the Internet, a fiber optic-based network, a cloud computing network, or the like, and/or a combination of these or other types of networks.

The number and arrangement of devices and networks shown in Fig. 2 are provided as an example. In practice, there may be additional devices and/or networks, fewer devices, different devices, or differently arranged devices than those shown in Fig. 2. Furthermore, two or more devices shown in Fig. 2 may be implemented within a single device, or a single device shown in Fig. 2 may be implemented as multiple, distributed devices. Additionally, or alternatively, a set of devices and/or networks (e.g., one or more devices) of environment 200 may perform one or more functions described as being performed by another set of devices and/or networks of environment 200.

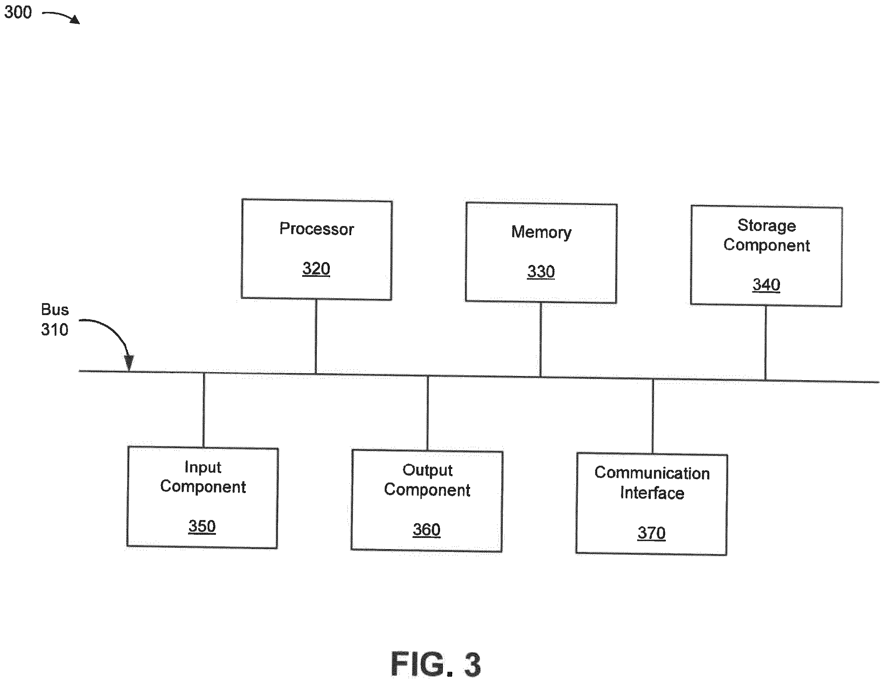

Fig. 3 is a diagram of example components of a device 300. Device 300 may correspond to sensor device 210, edge node 220, cloud server 230, computing resource 232, and/or user device 250. In some implementations, sensor device 210, edge node 220, cloud server 230, computing resource 232, and/or user device 250 may include one or more devices 300 and/or one or more components of device 300. As shown in Fig. 3, device 300 may include a bus 310, a processor 320, a memory 330, a storage component 340, an input component 350, an output component 360, and a communication interface 370.

Bus 310 includes a component that permits communication among the components of device 300. Processor 320 is implemented in hardware, firmware, or a combination of hardware and software. Processor 320 includes a central processing unit (CPU), a graphics processing unit (GPU), an accelerated processing unit (APU), a microprocessor, a microcontroller, a digital signal processor (DSP), a field-programmable gate array (FPGA), an application-specific integrated circuit (ASIC), or another type of processing component. In some implementations, processor 320 includes one or more processors capable of being programmed to perform a function. Memory 330 includes a random access memory (RAM), a read only memory (ROM), and/or another type of dynamic or static storage device (e.g., a flash memory, a magnetic memory, and/or an optical memory) that stores information and/or instructions for use by processor 320.

Storage component 340 stores information and/or software related to the operation and use of device 300. For example, storage component 340 may include a hard disk (e.g., a magnetic disk, an optical disk, a magneto-optic disk, and/or a solid state disk), a compact disc (CD), a digital versatile disc (DVD), a floppy disk, a cartridge, a magnetic tape, and/or another type of non-transitory computer-readable medium, along with a corresponding drive.

Input component 350 includes a component that permits device 300 to receive information, such as via user input (e.g., a touch screen display, a keyboard, a keypad, a mouse, a button, a switch, and/or a microphone). Additionally, or alternatively, input component 350 may include a sensor for sensing information (e.g., a global positioning system (GPS) component, an accelerometer, a gyroscope, and/or an actuator). Output component 360 includes a component that provides output information from device 300 (e.g., a display, a speaker, and/or one or more light-emitting diodes (LEDs)).

Communication interface 370 includes a transceiver-like component (e.g., a transceiver and/or a separate receiver and transmitter) that enables device 300 to communicate with other devices, such as via a wired connection, a wireless connection, or a combination of wired and wireless connections. Communication interface 370 may permit device 300 to receive information from another device and/or provide information to another device. For example, communication interface 370 may include an Ethernet interface, an optical interface, a coaxial interface, an infrared interface, a radio frequency (RF) interface, a universal serial bus (USB) interface, a Wi-Fi interface, a cellular network interface, or the like.

Device 300 may perform one or more processes described herein. Device 300 may perform these processes in response to processor 320 executing software instructions stored by a non-transitory computer-readable medium, such as memory 330 and/or storage component 340. A computer-readable medium is defined herein as a non-transitory memory device. A memory device includes memory space within a single physical storage device or memory space spread across multiple physical storage devices.

Software instructions may be read into memory 330 and/or storage component 340 from another computer-readable medium or from another device via communication interface 370. When executed, software instructions stored in memory 330 and/or storage component 340 may cause processor 320 to perform one or more processes described herein. Additionally, or alternatively, hardwired circuitry may be used in place of or in combination with software instructions to perform one or more processes described herein. Thus, implementations described herein are not limited to any specific combination of hardware circuitry and software.

The number and arrangement of components shown in Fig. 3 are provided as an example. In practice, device 300 may include additional components, fewer components, different components, or differently arranged components than those shown in Fig. 3. Additionally, or alternatively, a set of components (e.g., one or more components) of device 300 may perform one or more functions described as being performed by another set of components of device 300.

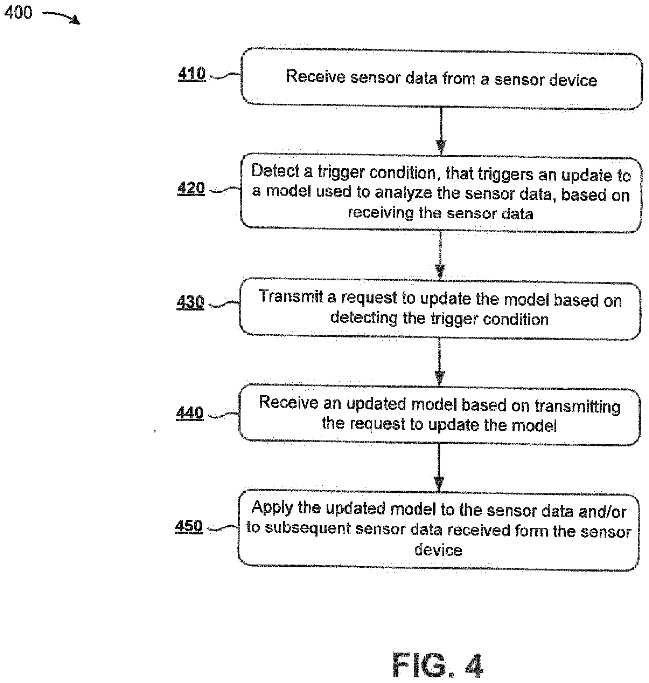

Fig. 4 is a flow chart of an example process 400 for dynamically updating a model based on detecting a trigger condition. In some implementations, one or more process blocks of Fig. 4 may be performed by edge node 220. In some implementations, one or more process blocks of Fig. 4 may be performed by another device or a group of devices separate from or including edge node 220, such as sensor device 210 and/or cloud server 230.

As shown in Fig. 4, process 400 may include receiving sensor data from a sensor device (block 410). For example, edge node 220 may receive, from sensor device 210, sensor data as part of a decentralized internet of things (IoT) system. The sensor data may include information that identifies a parameter (e.g., a parameter of a person, such as a heart rate or a blood sugar level; a parameter of a physical environment, such as a humidity level or an amount of sunlight; a parameter such as a frequency at which the sensor data is to be measured; a parameter relating to an amount of sensor data to be received at one time; a parameter of a device; a parameter relating to a time of delivery of the sensor data; etc.), information that identifies an entity associated with the sensor data (e.g., a patient receiving a heart rate measurement, an owner of a device being monitored, etc.), information that identifies a device associated with the sensor data (e.g., a device identifier that identifies sensor device 210), a time stamp associated with the sensor data, or the like. In some implementations, sensor data may include physiological data associated with a person.

In some implementations, edge node 220 may receive sensor data as part of a decentralized IoT system. For example, the decentralized IoT system may include one or more devices (e.g., one or more sensor devices 210, one or more edge nodes 220, one or more cloud servers 230, etc.) to dynamically detect, measure, analyze, and transmit sensor data in particular environments. As an example, sensor device 210 may transmit sensor data to edge node 220, and edge node 220 may apply a model to analyze the sensor data in a particular manner. In this case, edge node 220 may transmit the sensor data and/or the results of analyzing the sensor data to cloud server 230. Additionally, or alternatively, edge node 220 may transmit the sensor data to user device 250, and user device 250 may transmit the sensor data to cloud server 230.

As further shown in Fig. 4, process 400 may include detecting a trigger condition, that triggers an update to a model used to analyze the sensor data, based on receiving the sensor data (block 420). For example, edge node 220 may detect a trigger condition that triggers an update to a model. The trigger condition may include, for example, a change in a parameter associated with sensor device 210, the sensor data, and/or the delivery of the sensor data, a change in an entity being monitored in association with the sensor data, and/or the like. In some cases, the model that edge node 220 applies to the sensor data may analyze the sensor data in a particular manner (e.g., the model may be configured to analyze a body temperature), and the change in the sensor data may cause the model to be unable to analyze the sensor data (e.g., the model may not be configured to analyze a heart rate).

In some implementations, edge node 220 may detect a trigger condition by detecting a change in a type of data (e.g., a parameter) included in the sensor data. For example, edge node 220 may compare a type of data included in the sensor data (e.g., a heart rate level, a glucose level, etc.) and one or more types of data associated with a model (e.g., a heart rate level, a glucose level, etc.), and edge node 220 may detect the change in the type of data based on the comparison. As an example, edge node 220 may receive sensor data that includes a type of data associated with a heart rate. In this case, if edge node 220 is applying a model to monitor a different type of data, such as a glucose level, then edge node 220 may determine that the trigger condition is detected. Additionally, or alternatively, edge node 220 may detect a change in a type of data included in the sensor data when a type of data is removed from the sensor data.

Additionally, or alternatively, edge node 220 may detect a trigger condition by detecting a change in a frequency (e.g., a parameter) with which the sensor data is received. For example, edge node 220 may determine a time interval associated with receiving the sensor data, and may determine that the time interval satisfies a threshold. In this case, edge node 220 may detect the change in the frequency based on determining that the time interval satisfies the threshold. In some cases, edge node 220 may determine an average interval over a period of time, and if an interval between consecutively received sensor data falls outside a threshold range of the average interval, the trigger condition may be detected.

Additionally, or alternatively, edge node 220 may detect a trigger condition by detecting a change in an amount of sensor data (e.g., a parameter) to be received from sensor device 210 at one time. For example, edge node 220 may apply a model that is configured to receive a particular amount (e.g., in bytes) of sensor data at one time, and may determine that the particular amount of sensor data received from sensor device 210 satisfies a threshold. In this case, edge node 220 may detect the change in the particular amount of sensor data based on determining that the particular amount of sensor data satisfies the threshold.

Additionally, or alternatively, edge node 220 may detect a trigger condition by detecting a change in a time of delivery (e.g., a parameter) of the sensor data. For example, edge node 220 may apply a model that is configured to receive sensor data at a particular time (e.g., an interval of five minutes), and may determine that the sensor data is received at a different time by comparing time stamps associated with incoming sensor data. As an example, edge node 220 may determine an interval associated with receiving the sensor data by comparing a time stamp for a first transmission of sensor data and a time stamp for a second transmission of sensor data, and may detect the change in the time of delivery of the sensor data based on determining that the interval is different than the particular time that the model is configured to receive sensor data.

Additionally, or alternatively, edge node 220 may detect a trigger condition by detecting a change in an entity being monitored in association with the sensor data. For example, edge node 220 may compare a first entity identifier, that identifies the entity being monitored in association with the sensor data, and a second entity identifier associated with the model. In this case, edge node 220 may determine that the first entity identifier is different from the second entity identifier, and may detect the change in the entity based on determining that the first entity identifier is different from the second entity identifier. As an example, edge node 220 may receive sensor data that includes a metadata tag indicating that the sensor data is associated with entity A (e.g., a person, such as a patient at a hospital, a machine, a geographic location, an object, etc.). In this case, if the entity associated with the model is entity B, then edge node 220 may determine that the trigger condition is detected.

In some implementations, edge node 220 may detect a trigger condition associated with a combination of parameters and/or entities. For example, edge node 220 may detect a trigger condition by detecting a change in two or more parameters and/or entities, and may detect the changes in the same manner described above.

By detecting a trigger condition, edge node 220 identifies situations where the model may be insufficient to analyze the sensor data, allowing edge node 220 to conserve processing resources and/or memory resources. For example, edge node 220 may conserve processing resources and/or memory resources by not analyzing sensor data that the model is unable to properly process, and may make efficient use of overall network resources by transmitting a request to cloud server 230 to update the model, as described further herein. Additionally, or alternatively, edge node 220 identifies situations where the model may analyze sensor data inaccurately, and requests an update to the model so that edge node 220 can accurately analyze the sensor data.

Additionally, or alternatively, edge node 220 may determine whether a change associated with the trigger condition complies with a set of domain-specific rules. For example, the set of domain-specific rules may identify a range of acceptable values for a parameter, and if the sensor data includes a value for the parameter that is outside of the acceptable range, then a model update is not triggered, despite the trigger condition being satisfied.

In some cases, edge node 220 may identify a domain-specific rule associated with a domain, and may determine whether the sensor data satisfies the domain-specific rule. For example, edge node 220 may determine a domain associated with the sensor data, and may identify a domain-specific rule associated with the domain, by accessing information included in the sensor data (e.g., a domain identifier, a domain-specific rule associated with the domain identifier, etc.). Edge node 220 may determine whether the sensor data satisfies the domain-specific rule by comparing the sensor data and the domain-specific rule to determine if the parameter is within an acceptable range of values associated with the domain-specific rule. If the sensor data is within the acceptable range of values associated with the domain-specific rule, edge node 220 may transmit a request to update the model based on determining that the sensor data satisfies the domain-specific rule. If the sensor data is not within the acceptable range of values associated with the domain-specific rule, edge node 220 may not transmit the request to update the model, even if the trigger condition is otherwise satisfied.

By determining whether the sensor data satisfies a set of domain-specific rules, edge node 220 conserves processing resources and network resources that may otherwise be used to request and update the model based on inaccurate sensor data.

As an example, assume that edge node 220 uses a model to monitor body temperature of a first patient in a hospital. In this case, assume that edge node 220 receives, from sensor device 210, sensor data that includes a type of data associated with a body temperature for a second patient in the hospital. Further assume that edge node 220 stores a domain-specific rule for monitoring body temperature, which indicates that a human body temperature must be between 90 degrees Fahrenheit and 115 degrees Fahrenheit. However, if the sensor data indicates a body temperature level of 120 degrees Fahrenheit, edge node 220 may interpret this sensor data as an error and may not request to update the model (despite the trigger condition being satisfied due to receiving sensor data associated with the second patient).

As further shown in Fig. 4, process 400 may include transmitting a request to update the model based on detecting the trigger condition (block 430). For example, the detection of the trigger condition may indicate to edge node 220 that the model lacks the configuration needed to properly analyze the sensor data, and as a result, edge node 220 may transmit, to cloud server 230, a request to update the model (to obtain an update to the model).

In some implementations, edge node 220 may modify (e.g., increase, decrease, etc.) a frequency with which the sensor data is transmitted to cloud server 230. For example, edge node 220 may automatically increase a frequency of transmission when edge node 220 requests to update a model. This may increase the rate at which cloud server 230 is able to receive the sensor data needed to update the model. In some cases, edge node 220 may wait until receiving an instruction from cloud server 230 to increase a frequency of transmission. Additionally, or alternatively, edge node 220 may modify the frequency with which the sensor data is received from sensor device 210. For example, edge node 220 may send, to sensor device 210, a request for sensor data, a request to increase the frequency with which sensor device 210 transmits sensor data, or the like. By requesting to update the model when the sensor data changes, edge node 220 provides for efficient use of network resources and cloud computing resources by utilizing cloud server 230 only when a task is required that edge node 220 may be unable to handle.

In some implementations, edge node 220 may cause the model to be updated based on detecting the trigger condition. For example, edge node 220 may detect the trigger condition, which may cause edge node 220 to update the model. In some cases, edge node 220 may determine that the trigger condition is associated with a change in a parameter that edge node 220 can update (e.g., a change in a parameter that can be updated without using extensive processing resources). In other cases, a change in a parameter may require multiple actions to update the model, and edge node 220 may perform some of the actions (e.g., actions that may not require extensive use of processing resources), while transmitting a request to update the model to cloud server 230 to perform other actions (e.g., actions that may require extensive use of processing resources).

As further shown in Fig. 4, process 400 may include receiving an updated model based on transmitting the request to update the model (block 440). For example, edge node 220 may receive, from cloud server 230, the updated model that allows edge node 220 to analyze sensor data associated with the trigger condition. In this case, cloud server 230 may update (or generate) the model by receiving additional sensor data associated with the trigger condition, and analyzing the additional sensor data based on historical data and/or collaborative data, a set of domain-specific rules, a training window, and/or the like, and may transmit the updated model to edge node 220.

In some implementations, cloud server 230 may update the model based on historical data and/or collaborative data. Historical data may include past sensor data associated with a particular entity (e.g., a particular patient associated with the model being updated), and collaborative data may include past sensor data associated with different entities (e.g., not associated with the model being updated) that share one or more characteristics with the particular entity (e.g., an entity that shares a similar type of measured data, an entity that shares a similar age, background, demographics, etc.). In some cases, cloud server 230 may query a data structure for historical data and/or collaborative data, and may update the model using the query search results. In this case, cloud server 230 may use information included in the sensor data as the search query term (e.g., an entity identifier), and cloud server 230 may search the data structure for corresponding information (e.g., a corresponding entity identifier) that may indicate that the entity has historical data for the type of data being measured. If cloud server 230 determines that the entity has historical data for the type of data being measured, the historical data may be used to update the model.

If cloud server 230 determines that the entity does not have historical data for the type of data being measured, the collaborative data may be used to update the model. In this case, cloud server 230 may use information included in the sensor data as the search query term (e.g., information regarding one or more characteristics), and cloud server 230 may search the data structure for corresponding information (e.g., information regarding one or more corresponding characteristics) that may indicate that there is historical data and/or collaborative data for the type of data being measured. Cloud server 230 may use the historical data and/or the collaborative data to update the model.

Additionally, or alternatively, cloud server 230 may update the model using the set of domain-specific rules. For example, the set of domain-specific rules may allow cloud server 230 to update the model by interpreting the sensor data in a particular manner. In some cases, cloud server 230 may apply the set of domain-specific rules to the sensor data, which may allow cloud server 230 to interpret the sensor data in different ways, depending on context.

As an example, assume edge node 220 applies a model that is configured to analyze body temperature, and that edge node 220 receives sensor data that includes a type of data relating to measuring glucose levels, and a type of data relating to an amount of time that has passed since a patient's last meal. Further assume that this satisfies the trigger condition, and that edge node 220 transmits a request to update the model. In this case, cloud server 230 may apply a set of domain-specific rules relating to glucose levels to the sensor data, and may classify a glucose level associated with the sensor data as postprandial blood sugar or fasting blood sugar based on the amount of time that has passed since the patient's last meal. Classifying glucose levels associated with the sensor data as postprandial blood sugar or fasting blood sugar allows cloud server 230 to accurately update the model based on context. By updating the model with the set of domain-specific rules, edge node 220 is able to properly analyze the sensor data within the context of that particular domain.

Additionally, or alternatively, edge node 220 may receive, from cloud server 230, information identifying a training window. The training window may indicate a frequency with which the sensor data is to be transmitted to cloud server 230 and/or a length of time that sensor data is to be transmitted to cloud server 230. For example, cloud server 230 may determine the training window by monitoring a frequency at which requests to update the model are received. By training the model on the cloud server 230 (e.g., using an optimized training window and/or constraint satisfaction), edge node(s) 220 may conserve processing resources that would otherwise be used to train the model, thereby making edge node(s) 220 capable of analyzing large volumes of sensor data for fog computing.

In some cases, sensor data may change frequently, leading to a high frequency of requests to update the model. This may be an indicator that a shorter training window is needed, because edge node 220 may need to apply the updated model before a subsequent change to the sensor data is detected. In other cases, sensor data may change infrequently, leading to a low frequency of requests to update the model. This may be an indicator that a larger training window may be used, which may conserve network resources by allowing edge node 22 to wait until a period of low network traffic to transmit the sensor data needed to update the model. While cloud server 230 is waiting for the sensor data needed to update the model, edge node 220 may store the sensor data, even if edge node 220 lacks the updated model needed to properly analyze the sensor data. When edge node 220 receives the training window, edge node 220 may set the frequency with which the sensor data is to be transmitted to cloud server 230 to the training window, and/or the length of time that sensor data is to be transmitted to cloud server 230.

By receiving the updated model based on historical data, collaborative data, a set of domain-specific rules, and/or a training window, edge node 220 is able to properly analyze the sensor data.

As further shown in Fig. 4, process 400 may include applying the updated model to the sensor data and/or to subsequent sensor data received from the sensor device or another sensor device (block 450). For example, edge node 220 may apply the updated model to the sensor data and/or to the subsequent sensor data received from one or more sensor devices 210 (e.g., sensor device 210, or another sensor device 210 if the trigger condition is associated with a change in sensor device), which may allow edge node 220 to properly analyze the sensor data. In this case, after receiving the updated model, edge node 220 may automatically decrease a frequency at which the data is to be transmitted to cloud server 230. By automatically decreasing the frequency at which the sensor data is transmitted to cloud server 230 when the updated model is received, edge node 220 conserves processing resources by only transmitting data at a high frequency when a model needs to be updated.

In some implementations, edge node 220 may perform one or more actions based on applying the model. For example, edge node 220 may perform an action by analyzing the sensor data associated with the updated model, and may provide the analyzed sensor data to user device 250. For example, in certain domains (e.g., medical, industrial etc.), it may be advantageous to provide the sensor data to user device 250 for a human analysis. As an example, edge node 220 may provide sensor data associated with a medical-related parameter (e.g., a heart rate measurement) to user device 250 (e.g., a Doctor's computer), and this may allow a user (e.g., the Doctor) to determine whether the medical-related parameter associated with the sensor data is in a normal condition or a critical condition.

Additionally, or alternatively, edge node 220 may perform an action by updating one or more sensor devices 210. For example, edge node 220 may update a sensor device 210 by detecting an error associated with the sensor data and providing an instruction (e.g., to sensor device 210, to user device 250, to a domain-specific device, to a different device, etc.) to cause sensor device 210 to recalibrate, reboot, power down, and/or the like and/or to cause another sensor device 210 to power up, to be calibrated, to be configured, and/or the like. As an example, edge node 220 may determine that the sensor data satisfies an error threshold, and may provide an instruction to user device 250 based on determining that the sensor data satisfies the error threshold.

Additionally, or alternatively, edge node 220 may perform an action by analyzing the sensor data associated with the updated model, and may automatically provide an instruction, to a domain-specific machine, based on the analyzed sensor data. For example, edge node 220 may be configured to automatically provide an instruction to a domain-specific machine when a condition associated with the sensor data is satisfied. As an example, edge node 220 may analyze sensor data associated with an updated model, and the sensor data may include a data type relating to a heart rate measurement. In this case, edge node 220 may be configured to automatically provide an instruction (e.g., an alert) to a life support system, or a related medical device, if the condition associated with the heart rate measurement is satisfied.

In some implementations, cloud server 230 may transmit a command that overrides an instruction and/or information provided by an edge node 220 to a sensor device 210. For example, edge node 220 may generate an instruction that is outdated or inaccurate (e.g., because edge node 220 does not have a holistic view of data, whereas cloud server 230 does). In this case, cloud server 230 may receive the instruction from edge node 220, may determine that the instruction is outdate or inaccurate (e.g., by analyzing data stored by cloud server 230), and may transmit a command to override the instruction. In this case, edge node 220 may receive the command from cloud server 230, and may provide the command to sensor device 210.

Although Fig. 4 shows example blocks of process 400, in some implementations, process 400 may include additional blocks, fewer blocks, different blocks, or differently arranged blocks than those depicted in Fig. 4. Additionally, or alternatively, two or more of the blocks of process 400 may be performed in parallel. While process 400 may have referred to one or more sensor devices 210 associated with an entity, in practice, implementations described herein may include multiple sensor devices associated with each entity, a single sensor device 210 associated with multiple entities, and/or the like.

By dynamically updating a model based on changes to the sensor data, edge node 220 efficiently utilizes network resources. For example, if edge node 220 does not detect a change in the sensor data, edge node 220 may use processing resources and/or memory resources to analyze the sensor data. However, if edge node 220 does detect a change in the sensor data, edge node 220 may conserve processing resources and/or memory resources and transmit a request to cloud server 230 to update the model. Furthermore, edge node 220 conserves network resources and/or cloud computing resources by not requesting an unnecessary update to the model. Furthermore, in the case of physiological data, dynamically updating the model may permit real-time adaptation to rapidly changing patient conditions, may permit compliance with changing diagnostic requirements, and/or the like.

The foregoing disclosure provides illustration and description, but is not intended to be exhaustive or to limit the implementations to the precise form disclosed. Modifications and variations are possible in light of the above disclosure or may be acquired from practice of the implementations.

As used herein, the term component is intended to be broadly construed as hardware, firmware, and/or a combination of hardware and software.

Some implementations are described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, etc.

It will be apparent that systems and/or methods, described herein, may be implemented in different forms of hardware, firmware, or a combination of hardware and software. The actual specialized control hardware or software code used to implement these systems and/or methods is not limiting of the implementations. Thus, the operation and behavior of the systems and/or methods were described herein without reference to specific software code-it being understood that software and hardware can be designed to implement the systems and/or methods based on the description herein.

Even though particular combinations of features are recited in the claims and/or disclosed in the specification, these combinations are not intended to limit the disclosure of possible implementations. In fact, many of these features may be combined in ways not specifically recited in the claims and/or disclosed in the specification. Although each dependent claim listed below may directly depend on only one claim, the disclosure of possible implementations includes each dependent claim in combination with every other claim in the claim set.

No element, act, or instruction used herein should be construed as critical or essential unless explicitly described as such. Also, as used herein, the articles "a" and "an" are intended to include one or more items, and may be used interchangeably with "one or more." Furthermore, as used herein, the term "set" is intended to include one or more items (e.g., related items, unrelated items, a combination of related and unrelated items, etc.), and may be used interchangeably with "one or more." Where only one item is intended, the term "one" or similar language is used. Also, as used herein, the terms "has," "have," "having," or the like are intended to be open-ended terms. Further, the phrase "based on" is intended to mean "based, at least in part, on" unless explicitly stated otherwise.

## Claims

1. A device (220), comprising:
one or more processors adapted to:
receive physiological data from one or more sensor devices (210), the device applying a model to analyze the physiological data;
detect a trigger condition, that triggers an update to the model used to analyze the physiological data, based on receiving the physiological data,
the trigger condition including at least one of:
a change in a type of data included in the physiological data,
a change in an entity being monitored in association with the physiological data, or
a change in a frequency with which the physiological data is received from the one or more sensor devices, and wherein detecting the at least one trigger condition allows the device (220) to identify that the model is insufficient to analyze the physiological data;
transmit to a cloud computing device (230) a request to update the model to account for the change in the type of data, the change in the entity, or the change in the frequency based on detecting the trigger condition;
receive from the cloud computing device (230) an updated model, that accounts for the change in the type of data, the change in the entity, or the change in the frequency based on transmitting the request to update the model; and
analyze the physiological data and/or subsequent physiological data received from the one or more sensor devices (210) using the updated model.

2. The device (220) of claim 1, where the one or more processors, when detecting the trigger condition, are adapted to:
compare a type of data included in the physiological data and one or more types of data associated with the model;
determine that the type of data is not included in the one or more types of data; and
detect the change in the type of data based on determining that the type of data is not included in the one or more types of data.

3. The device (220) of any one of the preceding claims, where the one or more processors, when detecting the trigger condition, are adapted to
compare a first entity identifier, that identifies the entity being monitored in association with the physiological data, and a second entity identifier associated with the model;
determine that the first entity identifier is different from the second entity identifier; and
detect the change in the entity based on determining that the first entity identifier is different from the second entity identifier.

4. The device (220) of any one of the preceding claims, where the one or more processors, when detecting the trigger condition, are adapted to:
determine a time interval associated with receiving the physiological data; determine that the time interval satisfies a threshold; and
detect the change in the frequency based on determining that the time interval satisfies the threshold.

5. The device (220) of any one of the preceding claims, where the one or more processors are further adapted to:
determine a domain associated with the physiological data;
identify a domain-specific rule associated with the domain;
determine that the physiological data satisfies the domain-specific rule; and
where the one or more processors, when transmitting the request, are to:
transmit the request based on determining that the physiological data satisfies the domain-specific rule.

6. The device (220) of any one of the preceding claims, where the one or more processors are further adapted to transmit sensor data to a cloud server; and modify a frequency with which the physiological data is transmitted to the cloud server.

7. The device (220) of any one of the preceding claims, where the one or more processors are further adapted to:
receive information identifying a training window,
the training window indicating a frequency with which additional physiological data is to be transmitted to the cloud computing device, to update the model; and
transmit the additional physiological data to the cloud computing device, to update the model, based on the training window.

8. A method, comprising:
receiving, by a device (220), physiological data from a sensor device (210), the device applying a model to analyze the physiological data;
detecting, by the device (220), a trigger condition that triggers an update to the model, based on receiving the physiological data,
the trigger condition including at least one of:
a change in a type of data included in the physiological data,
a change in a frequency with which the physiological data is received from the sensor device, or
a change in an entity being monitored in association with the physiological data, or
a change in an amount of physiological data, or
a change in a time of delivery of the physiological data,
and wherein detecting the at least one trigger condition allows the device (220) to
identify that the model is insufficient to analyze the physiological data;
causing, by the device (220), the model to be updated to account for the change in the type of data, the change in the frequency, or the change in the entity based on detecting the trigger condition;
transmitting, by the device (220), to a cloud computing device (230) a request to update the model to account for the change in the type of data, the change in the entity, or the change in the frequency based on detecting the trigger condition;
receiving, by the device (220), from the cloud computing device (230) an updated model, that accounts for the change in the type of data, the change in the entity, or the change in the frequency based on transmitting the request to update the model; and
analyzing, by the device (220), the physiological data and/or subsequent physiological data using the updated model; and
performing, by the device (220), one or more actions based on analyzing the physiological data and/or the subsequent physiological data.

9. The method of claim 8 where performing the one or more actions comprises:
providing the physiological data and/or the subsequent physiological data to a user device.

10. The method of any one of claims 8 or 9 where performing the one or more actions comprises:
updating the sensor device (210).

11. The method of any one of claims 8-10, where performing the one or more actions comprises:
determining that the physiological data and/or the subsequent physiological data satisfies a threshold; and
providing an instruction, to a domain-specific device, based on determining that the physiological data and/or the subsequent physiological data satisfies the threshold.

12. The method of any one of claims 8-11, where the device (220) is an edge node in a distributed computing environment, and wherein the cloud computing device is included in the distributed computing environment.

13. The method of any one of claims 8-12, where the physiological data is received from a plurality of sensor devices (210) associated with the entity, the plurality of sensor devices including the sensor device.

14. A computer program product comprising machine executable instructions residing on non-transitory computer readable media, which, when loaded into a device according to any one of claims 1 to 7, and said instructions are executed by the one ore more processors, cause the device to perform operations according to the method of any one of claims 8-13.

## Patentansprüche

1. Eine Vorrichtung (220), die Folgendes umfasst:
einen oder mehrere Prozessoren, die eingerichtet sind, um Folgendes zu bewerkstelligen:
Empfangen von physiologischen Daten, die von einem oder mehreren Sensorgeräten (210) ausgehen, wobei die Vorrichtung ein Modell anwendet, um die physiologischen Daten zu analysieren;
Detektieren einer Auslösebedingung, die eine Aktualisierung des Modells auslöst, das zum Analysieren der physiologischen Daten verwendet wird, basierend auf dem Empfang der physiologischen Daten,
wobei die Auslösebedingung mindestens eine der folgenden beinhaltet:
eine Änderung eines Typs von Daten, die in den physiologischen Daten enthalten sind,
eine Änderung einer Entität, die in Verbindung mit den physiologischen Daten überwacht wird, oder
eine Änderung einer Frequenz, mit der die physiologischen Daten von der einen oder den mehreren Sensorvorrichtungen empfangen werden, und wobei das Erfassen der mindestens einen Auslösebedingung es der Vorrichtung (220) ermöglicht, zu erkennen, dass das Modell nicht ausreicht, um die physiologischen Daten zu analysieren;
Senden, an eine *Cloud-Computing*-Vorrichtung (230), einer Anforderung, das Modell zu aktualisieren, zur Berücksichtigung der Änderung des Datentyps, der Änderung der Entität oder der Änderung der Frequenz basierend auf dem Erfassen der Auslösebedingung;
Empfangen, von der *Cloud-Computing*-Vorrichtung (230), eines aktualisierten Modells, das die Änderung des Datentyps, die Änderung der Entität oder die Änderung der Frequenz basierend auf dem Senden der Anforderung zur Aktualisierung des Modells berücksichtigt; und
Analysieren der physiologischen Daten und/oder der nachfolgenden physiologischen Daten, die von dem einen oder den mehreren Sensorgeräten (210) ausgehend, empfangen werden, unter Verwendung des aktualisierten Modells.

2. Die Vorrichtung (220) nach Anspruch 1, wobei der eine oder die mehreren Prozessoren, beim Erfassen der Auslösebedingung, dazu ausgelegt sind, Folgendes zu bewerkstelligen:
Vergleichen eines Datentyps, der in den physiologischen Daten enthalten ist, mit einen oder mehreren Datentypen, die mit dem Modell verknüpft sind,
Feststellen, dass der Datentyp nicht in dem einen oder den mehreren Datentypen enthalten ist; und
Erkennen der Änderung des Datentyps, basierend auf der Feststellung, dass der Datentyp nicht in dem einen oder den mehreren Datentypen enthalten ist.

3. Die Vorrichtung (220) nach irgendeinem der vorstehenden Ansprüche, wobei der eine oder die mehreren Prozessoren beim Erfassen der Auslösebedingung eingerichtet sind, um Folgendes zu bewerkstelligen:
Vergleichen eines ersten Entitätsidentifikators, der die überwachte Entität in Verbindung mit den physiologischen Daten identifiziert, mit einem zweiten Entitätsidentifikator, der mit dem Modell verknüpft ist,
Feststellen, dass der erste Entitätsidentifikator sich vom zweiten Entitätsidentifikator unterscheidet; und
Erkennen der Änderung der Entität basierend auf der Feststellung, dass der erste Entitätsbezeichner sich vom zweiten Entitätsbezeichner unterscheidet.

4. Die Vorrichtung (220) nach irgendeinem der vorstehenden Ansprüche, wobei der eine oder die mehreren Prozessoren beim Erfassen der Auslösebedingung eingerichtet sind, um Folgendes zu bewerkstelligen:
Bestimmen eines Zeitintervalls, das mit dem Empfangen der physiologischen Daten verknüpft ist;
Feststellen, dass das Zeitintervall einen Schwellenwert erfüllt; und
Erkennen der Änderung der Frequenz basierend auf der Feststellung, dass das Zeitintervall den Schwellenwert erfüllt.

5. Die Vorrichtung (220) nach irgendeinem der vorstehenden Ansprüche, wobei der eine oder die mehreren Prozessoren ferner eingerichtet sind, um Folgendes zu bewerkstelligen:
Bestimmen einer Domäne (*domain*), die mit den physiologischen Daten assoziiert ist;
Identifizieren einer domänenspezifischen Regel, die mit der Domäne assoziiert ist;
Feststellen, dass die physiologischen Daten die domänenspezifische Regel erfüllen; und
wobei der eine oder die mehreren Prozessoren, wenn sie die Anforderung senden, dazu ausgebildet sind, um Folgendes zu bewerkstelligen:
Senden der Anforderung basierend auf der Feststellung, dass die physiologischen Daten die domänenspezifische Regel erfüllen.

6. Die Vorrichtung (220) nach irgendeinem der vorstehenden Ansprüche, wobei der eine oder die mehreren Prozessoren ferner dazu eingerichtet sind, Sensordaten an einen Cloud-Server zu senden; und
eine Frequenz zu modifizieren, mit der die physiologischen Daten an den Cloud-Server gesendet werden.

7. Die Vorrichtung (220) nach irgendeinem der vorstehenden Ansprüche, wobei der eine oder die mehreren Prozessoren ferner eingerichtet sind, um Folgendes zu bewerkstelligen:
Empfangen von Informationen, die ein Trainingsfenster identifizieren, wobei das Trainingsfenster eine Frequenz angibt, mit der zusätzliche physiologische Daten an die *Cloud-Computing*-Vorrichtung gesendet werden sollen, um das Modell zu aktualisieren; und
Senden der zusätzlichen physiologischen Daten an die *Cloud-Computing-*Vorrichtung, um das Modell zu aktualisieren, basierend auf dem Trainingsfenster.

8. Ein Verfahren, das Folgendes umfasst:
Empfangen, durch eine Vorrichtung (220), physiologischer Daten von einer Sensorvorrichtung (210), wobei die Vorrichtung ein Modell anwendet, um die physiologischen Daten zu analysieren;
Erfassen, durch die Vorrichtung (220), einer Auslösebedingung, die eine Aktualisierung des Modells auslöst, basierend auf dem Empfang der physiologischen Daten,
wobei die Auslösebedingung mindestens eine der folgenden beinhaltet:
eine Änderung eines Typs von Daten, die in den physiologischen Daten enthalten sind,
eine Änderung einer Frequenz, mit der die physiologischen Daten von der Sensorvorrichtung empfangen werden, oder
eine Änderung einer Entität, die in Verbindung mit den physiologischen Daten überwacht wird, oder
eine Änderung der Menge der physiologischen Daten, oder
eine Änderung in einer Zeit der Übermittlung der physiologischen Daten,
und wobei das Erfassen der mindestens einen Auslösebedingung es der Vorrichtung (220) ermöglicht, Folgendes zu bewerkstelligen:
Erkennen, dass das Modell unzureichend ist, um die physiologischen Daten zu analysieren;
Veranlassen, durch die Vorrichtung (220), dass das Modell aktualisiert wird, um die Änderung des Datentyps, die Änderung der Frequenz oder die Änderung der Entität basierend auf dem Erfassen der Auslösebedingung zu berücksichtigen;
Senden, durch die Vorrichtung (220), einer Anforderung an eine *Cloud-Computing*-Vorrichtung (230), das Modell zu aktualisieren, um die Änderung des Datentyps, die Änderung der Entität oder die Änderung der Frequenz basierend auf dem Erfassen der Auslösebedingung zu berücksichtigen;
Empfangen, durch die Vorrichtung (220) und ausgehend von der *Cloud-Computing*-Vorrichtung (230), eines aktualisierten Modells, das die Änderung des Datentyps, die Änderung der Entität oder die Änderung der Frequenz basierend auf dem Übertragen der Anforderung zur Aktualisierung des Modells berücksichtigt; und
Analysieren, durch die Vorrichtung (220), der physiologischen Daten und/oder der nachfolgenden physiologischen Daten unter Verwendung des aktualisierten Modells; und
Durchführen, durch die Vorrichtung (220), einer oder mehrerer Aktionen basierend auf dem Analysieren der physiologischen Daten und/oder der nachfolgenden physiologischen Daten.

9. Das Verfahren nach Anspruch 8, wobei das Durchführen der einen oder mehreren Aktionen Folgendes umfasst:
Bereitstellen der physiologischen Daten und/oder der nachfolgenden physiologischen Daten für eine Benutzervorrichtung.

10. Das Verfahren nach irgendeinem der Ansprüche 8 oder 9, wobei das Durchführen der einen oder mehreren Aktionen Folgendes umfasst:
Aktualisieren der Sensorvorrichtung (210).

11. Das Verfahren nach irgendeinem der Ansprüche von 8 bis 10, wobei das Durchführen der einen oder mehreren Aktionen Folgendes umfasst:
Feststellen, dass die physiologischen Daten und/oder die nachfolgenden physiologischen Daten einen Schwellenwert erfüllen; und
Bereitstellen einer Anweisung, für eine domänenspezifische Vorrichtung, basierend auf der Feststellung, dass die physiologischen Daten und/oder die nachfolgenden physiologischen Daten den Schwellenwert erfüllen.

12. Das Verfahren nach irgendeinem der Ansprüche von 8 bis 11, wobei die Vorrichtung (220) ein Randknoten bzw. Edge-Knoten in einer verteilten Rechenumgebung ist, und wobei die *Cloud-Computing-*Vorrichtung in der verteilten Rechenumgebung enthalten ist.

13. Das Verfahren nach irgendeinem der Ansprüche von 8 bis 12, wobei die physiologischen Daten von einer Vielzahl von Sensorvorrichtungen (210) empfangen werden, die mit der Entität verknüpft sind, wobei die Vielzahl von Sensorvorrichtungen die Sensorvorrichtung beinhaltet.

14. Ein Computerprogrammprodukt, das maschinenausführbare Anweisungen umfasst, die sich auf nicht-flüchtigen computerlesbaren Medien befinden, die, wenn sie in eine Vorrichtung nach einem der Ansprüche 1 bis 7 geladen werden und die genannten Anweisungen von dem einen oder den mehreren Prozessoren ausgeführt werden, die Vorrichtung dazu veranlassen, Operationen entsprechend einem Verfahren nach irgendeinem der Ansprüche von 8 bis 13 durchzuführen.

## Revendications

1. Un dispositif (220), comprenant :
un ou plusieurs processeurs adaptés pour :
recevoir des données physiologiques provenant d'un ou de plusieurs dispositifs capteurs (210), le dispositif appliquant un modèle pour analyser les données physiologiques ;
détecter une condition de déclenchement, qui déclenche une mise à jour du modèle utilisé pour analyser les données physiologiques, sur la base de la réception des données physiologiques, la condition de déclenchement incluant au moins un des éléments suivants :
un changement d'un type de données inclus dans les données physiologiques,
un changement d'une entité surveillée en association avec les données physiologiques, ou
un changement de la fréquence à laquelle les données physiologiques sont reçues de la part d'un ou de plusieurs dispositifs de détection, et sachant que la détection d'au moins une condition de déclenchement permet au dispositif (220) d'identifier que le modèle est insuffisant pour analyser les données physiologiques ;
transmettre à un dispositif informatique en nuage (230) une demande de mise à jour du modèle pour prendre en compte le changement du type de données, le changement de l'entité ou le changement de la fréquence, sur la base de la détection de la condition de déclenchement ;
recevoir de la part du dispositif informatique en nuage (230) un modèle mis à jour, qui tient compte du changement du type de données, du changement de l'entité ou du changement de la fréquence, sur la base de la transmission de la demande de mise à jour du modèle ; et pour
analyser les données physiologiques et/ou les données physiologiques ultérieures reçues de la part d'un ou de plusieurs dispositifs capteurs (210) en utilisant le modèle mis à jour.

2. Le dispositif (220) d'après la revendication 1, sachant que le ou les processeurs, lors de la détection de la condition de déclenchement, sont adaptés pour :
comparer un type de données inclus dans les données physiologiques, et un ou plusieurs types de données associés au modèle ;
constater que le type de données n'est pas inclus dans le ou les types de données ; et pour
détecter le changement du type de données sur la base du constat que le type de données n'est pas inclus dans le ou les types de données.

3. Le dispositif (220) d'après l'une quelconque des revendications précédentes, sachant que le ou les processeurs, lors de la détection de la condition de déclenchement, sont adaptés pour :
comparer un premier identifiant d'entité, qui identifie l'entité surveillée en association avec les données physiologiques, et un deuxième identifiant d'entité associé au modèle ;
constater que le premier identifiant d'entité est différent du deuxième identifiant d'entité ; et pour
détecter le changement de l'entité sur la base du constat que le premier identifiant d'entité est différent du deuxième identifiant d'entité.

4. Le dispositif (220) d'après l'une quelconque des revendications précédentes, sachant que le ou les processeurs, lors de la détection de la condition de déclenchement, sont adaptés pour :
déterminer un intervalle de temps associé à la réception des données physiologiques ;
constater que l'intervalle de temps satisfait à un seuil ; et pour
détecter le changement de la fréquence sur la base du constat que l'intervalle de temps satisfait au seuil.

5. Le dispositif (220) d'après l'une quelconque des revendications précédentes, sachant que le ou les processeurs sont en outre adaptés pour :
déterminer un domaine associé aux données physiologiques ;
identifier une règle spécifique au domaine associée au domaine ;
constater que les données physiologiques satisfont à la règle spécifique au domaine ; et
sachant que le ou les processeurs, lorsqu'ils transmettent la demande, sont destinés à :
transmettre la demande sur la base du constat que les données physiologiques satisfont à la règle spécifique au domaine.

6. Le dispositif (220) d'après l'une quelconque des revendications précédentes, sachant que le ou les processeurs sont en outre adaptés pour transmettre des données de capteur à un serveur en nuage ; et pour
modifier une fréquence à laquelle les données physiologiques sont transmises au serveur en nuage.

7. Le dispositif (220) d'après l'une quelconque des revendications précédentes, sachant que l'un ou plusieurs processeurs sont en outre adaptés pour :
recevoir des informations identifiant une fenêtre d'apprentissage, la fenêtre d'apprentissage indiquant une fréquence à laquelle des données physiologiques supplémentaires doivent être transmises au dispositif d'informatique en nuage, pour mettre à jour le modèle ; et pour
transmettre les données physiologiques supplémentaires au dispositif informatique en nuage, afin de mettre à jour le modèle, sur la base de la fenêtre d'apprentissage.

8. Un procédé, comprenant le fait de :
recevoir, par un dispositif (220), des données physiologiques provenant d'un dispositif capteur (210), le dispositif appliquant un modèle pour analyser les données physiologiques ;
détecter, par le dispositif (220), une condition de déclenchement qui déclenche une mise à jour du modèle, sur la base de la réception des données physiologiques,
la condition de déclenchement incluant au moins un des éléments suivants:
un changement du type de données incluses dans les données physiologiques,
un changement de la fréquence à laquelle les données physiologiques sont reçues de la part du dispositif capteur, ou
un changement d'une entité surveillée en association avec les données physiologiques, ou
un changement de la quantité de données physiologiques, ou
un changement dans le temps de livraison des données physiologiques,
et sachant que la détection de l'au moins une condition de déclenchement permet au dispositif (220) de :
identifier que le modèle est insuffisant pour analyser les données physiologiques ;
provoquer, par le dispositif (220), la mise à jour du modèle pour tenir compte du changement du type de données, du changement de la fréquence ou du changement de l'entité sur la base de la détection de la condition de déclenchement ;
transmettre, par le dispositif (220), à un dispositif informatique en nuage (230) une demande de mise à jour du modèle pour prendre en compte le changement du type de données, le changement de l'entité, ou le changement de la fréquence sur la base de la détection de la condition de déclenchement ;
recevoir, par le dispositif (220), de la part du dispositif d'informatique en nuage (230), un modèle mis à jour, qui prend en compte le changement du type de données, le changement de l'entité, ou le changement de la fréquence sur la base de la transmission de la demande de mise à jour du modèle ; et de
analyser, par le dispositif (220), les données physiologiques et/ou les données physiologiques ultérieures en utilisant le modèle mis à jour ; et de
effectuer, par le dispositif (220), une ou plusieurs actions basées sur l'analyse des données physiologiques et/ou des données physiologiques ultérieures.

9. Le procédé d'après la revendication 8, sachant que le fait d'exécuter une ou plusieurs actions comprend le fait de :
fournir des données physiologiques et/ou des données physiologiques ultérieures à un dispositif utilisateur.

10. Le procédé d'après l'une quelconque des revendications 8 ou 9, sachant que le fait d'exécuter une ou plusieurs actions comprend le fait de :
mettre à jour le dispositif capteur (210).

11. Le procédé d'après l'une quelconque des revendications de 8 à 10, sachant que le fait d'exécuter une ou plusieurs actions comprend le fait de :
constater que les données physiologiques et/ou les données physiologiques ultérieures satisfont à un seuil ; et
fournir une instruction, à un dispositif spécifique au domaine, sur la base du constat que les données physiologiques et/ou les données physiologiques ultérieures satisfont au seuil.

12. Le procédé d'après l'une quelconque des revendications de 8 à 11, sachant que le dispositif (220) est un nœud de périphérie dans un environnement informatique distribué, et sachant que le dispositif informatique en nuage est inclus dans l'environnement informatique distribué.

13. Le procédé d'après l'une quelconque des revendications de 8 à 12, sachant que les données physiologiques sont reçues de la part d'une pluralité de dispositifs de détection (210) associés à l'entité, la pluralité de dispositifs de détection incluant le dispositif de détection.

14. Un produit programme informatique comprenant des instructions exécutables par machine résidant sur un support lisible par ordinateur non transitoire, qui, lorsqu'elles sont chargées dans un dispositif selon l'une quelconque des revendications de 1 à 7 et que lesdites instructions sont exécutées par le ou les processeurs, amènent le dispositif à exécuter des opérations conformes au procédé d'après l'une quelconque des revendications de 8 à 13.
